# EUROPÄISCHE PATENTANMELDUNG

(11) **EP 1 844 809 A1**
(43) Veröffentlichungstag der Anmeldung: **17.10.2007**
(21) Anmeldenummer: 06405163.4
(22) Anmeldetag: 13.04.2006
(51) Int. Cl.: A61M 15/00, A61J 1/03

(54) **Medikamentenmagazin für einen Inhalator, sowie Mehrdosispulverinhalator**

(71) Anmelder: Boehringer Ingelheim Pharma GmbH & Co. KG, 55216 Ingelheim am Rhein (DE)
(72) Erfinder: Wachtel, Herbert, 55218 Ingelheim am Rhein (DE); Geser, Johannes, 55218 Ingelheim am Rhein (DE); Metzger, Burkhard, 55218 Ingelheim am Rhein (DE); Spallek, Michael, 55218 Ingelheim am Rhein (DE); Krueger, Michael, 55218 Ingelheim am Rhein (DE); Kunze, Herbert, 44227 Dortmund (DE); Moser, Achim, 65843 Sulzbach (DE); Mock, Elmar, 2013 Colombier (CH); Lanci, Antonino, 3006 Bern (CH); Klopfenstein, André, 2520 La Neuveville (CH)
(74) Vertreter: Frei Patent Attorneys

(57) **Zusammenfassung**

Die Erfindung ist ein Medikamentenmagazin mit mindestens einer Medikamentenkammer zur Verwendung in einem Pulverinhalator, wobei das Magazin aus zwei aneinander angebrachten Folienbahnen gebildet ist, und zwischen den Folienbahnen die mindestens eine Medikamentenkammer ausgebildet ist. Die Medikamentenkammern weisen eine Innenstruktur (3) auf, welche in ihrem Inneren ein Volumen zur Aufnahme eines pulverförmigen Medikaments aufweist und dieses Innenvolumen gegenüber mechanischen Einflüssen von Aussen stabilisiert. In einer anderen Ausführungsform, in welcher die Innenstruktur vorzugsweise ebenfalls eine Stabilisierungsfunktion hat, weist die Struktur ein Mittel zum Öffnen der einen Folienbahn auf. Zudem beinhaltet die Medikamentenkammer ein Rückhaltemittel, welches die Innenstruktur nach einem Öffnen der Medikamentenkammer am Medikamentenmagazin hält.

## Beschreibung

Die Erfindung bezieht sich auf das Gebiet der Medikamentenmagazine, insbesondere auf ein Medikamentenmagazin für eine Inhalationsvorrichtung und einen Mehrdosispulverinhalator gemäss dem Oberbegriff der unabhängigen Patentansprüche.

Bei Medikamentenmagazinen stellt sich das Problem, wie eine Medikamentenkammer, in welcher sich ein Medikament befindet, geöffnet werden kann. Insbesondere für Medikamentenmagazine in Inhalatoren stellt sich die Anforderung, dass bei Medikamenten in Pulverform dieses weder bei der Lagerung noch beim Öffnen zusammengedrückt werden sollte, so dass keine Kompaktierung des Pulvers stattfindet. Zudem sollte ein Inhalator möglichst einfach, günstig und Platz sparend aufgebaut sein.

Es ist deshalb eine Aufgabe der Erfindung, ein Medikamentenmagazin für eine Inhalationsvorrichtung zu schaffen, welches einen verbesserten, insbesondere vereinfachten Öffnungsmechanismus in einem Inhalator ermöglicht.

Eine andere Aufgabe der Erfindung ist, ein Medikamentenmagazin zu schaffen, bei welchem ein im Magazin befindliches Pulver beim Öffnen der Kammer im wesentlichen nicht mechanisch belastet wird.

Diese Aufgaben lösen die Medikamentenmagazine mit den Merkmalen der entsprechenden unabhängigen Patentansprüche.
Bevorzugte Ausführungsformen sind in den abhängigen Ansprüchen definiert.

Das erfindungsgemässe Medikamentenmagazin ist zur Verwendung in einem Inhalationsgerät, vorzugsweise in einem Mehrdosispulverinhalator, gedacht und weist mindestens eine Medikamentenkammer auf. Das Magazin wird aus zwei aneinander angebrachten, beispielsweise aneinander gesiegelte Folienbahnen gebildet, wobei die mindestens eine Medikamentenkammer zwischen den Folienbahnen ausgebildet ist. In der Medikamentenkammer kann ein, ein Wirkstoff tragendes Pulver eingefüllt sein. Die Medikamentenkammer weist nun eine Innenstruktur auf, welche vorzugsweise ein separates Element, beispielsweise ein durch Spritzgiessen oder Thermoformen hergestelltes Kunststoffteil, ist.

In einer Ausführungsform der Erfindung weist die Innenstruktur an einer Vorderseite ein Mittel zum Öffnen der einen Folienbahn auf. Gleichzeitig umfasst die Medikamentenkammer auch ein Rückhaltemittel, welches die Innenstruktur nach einem Öffnen der Medikamentenkammer am Medikamentenmagazin hält und dafür sorgt, dass die Innenstruktur nicht heraus- oder abfällt und beispielsweise lose in einem Gerät herumliegt. Ein solches Rückhaltemittel wird bevorzugt durch ein Befestigen der Innenstruktur an einer Folie realisiert, oder auch durch Gestaltung der Innenstruktur, derart, dass diese beim bzw. nach einem Öffnen der Kammer an einer Folie oder Teilen davon ansteht.

In einer weiteren Ausführungsform der Erfindung, welche mit der ersten Ausführungsform kombiniert sein kann, weist die Innenstruktur in ihrem Inneren ein Volumen zur Aufnahme eines pulverförmigen Medikaments auf. Dabei wird durch die Innenstruktur dieses Innenvolumen oder auch die gesamte Medikamentenkammer gegenüber mechanischen Einflüssen von Aussen stabilisiert.

Das Medikamentenmagazin weist den Vorteil auf, dass auch in einem Folienblister ein Pulver vor einem Zusammendrücken und einem damit verbundenen Kompaktieren geschützt ist. Es können für die Herstellung des Folienblisters somit auch sehr dünne und flexible Folien verwendet werden. Dies hat wiederum auch auf den Speicherplatz in einem Inhalationsgerät Einfluss, dadurch dass bei gleichbleibenden Umfang ein Magazin mit einer grösseren Anzahl von Einzeldosen versehen werden kann, oder aber, dass ein Magazin kompakter und vielfältiger in einem Inhalator untergebracht werden kann. Je nach Ausführungsform kann eine Folie jedoch auch sehr stabil und fest sein und beispielsweise vorgängig eingebrachte Vertiefungen beinhalten zur Aufnahme der Innenstrukturen.

Auch können diese Innenstrukturen äussere Anstechmechanismen, wie Inhalationshohlnadeln unterstützen, in dem sie als Zentrier- und Anstechhilfe dienen und einen Blister dabei stablisieren. Die Innenstruktur kann dabei mit einem Öffnungsmittel, wie Stechpunkten oder Schneidkanten versehen sein, oder auch aufgrund der Scherfläche zum Öffnen einer Medikammentenkammer beitragen.

Einen Vorteil einer Innenstruktur, welche zum eigentlichen Öffnen einer Medikamentenkammer verwendet wird ist, dass kein äusserer Öffnungsmechnismus, wie beispielsweise eine Anstechnadel, eine Schneidkante etc., mehr als einmal verwendet wird. Dies trägt wesentlich zu einer hygienischeren Anwendung eines sogenannten Multidose-Gerätes bei. Es ist auch keine Zusatzrolle zum Aufrollen einer abgelösten Folie notwendig oder ein Zusatzreservoir für Abfälle, da ein Medikamentenmagazin vor und nach dem Gebrauch im wesentlichen denselben Umfang bzw. dieselbe Gestalt aufweist, einfach mit geöffneter, entleerter Kammer.

Auch ein Öffnungsmechanismus selber lässt sich einfacher gestalten, in dem im wesentlich ein Element vorhanden sein muss, welches Druck auf die Rückseite des Magazins im Bereich einer Medikamentenkammer ausübt. Je nach Gestaltung der Innenstruktur, ist ein Öffnen der Kammer auch durch ein einfaches Rollen über eine Rückseite einer Kammer möglich. Dies vereinfacht den Aufbau eines Inhalationsgerätes auch dadurch, dass ein solcher Druckmechanismus weniger genau als beispielsweise ein Anstechmechanismus sein muss. Diese Eigenschaften führen dazu, dass ein Inhalator entsprechend billiger und kleiner ausgeführt werden kann.

Es ist auch möglich Innenstrukturen zu schaffen, welche voneinander unabhängige Medikamenten-Einfüllöffnungen und Entnahmeöffnung aufweisen. Eine Einfüllöffnung ist bevorzugt gross und gegenüber einer anzubringenden Siegelfolie angeordnet. Ein oder mehrere Entnahmeöffnungen, welche nicht mit der Einfüllöffnung übereinstimmen, welche sich insbesondere auf einer anderen Seite der Innenstruktur befinden, bieten sehr vielfältige Möglichkeiten in Bezug auf beispielsweise den Aufbau eines Inhalationsgerätes, die Dosierung und Beschaffenheit eines Medikamentes etc..

In einer bevorzugten Ausführungsform wird eine Innenstruktur durch Spritzgiessen und vorzugsweise einstückig hergestellt. Es ist jedoch auch möglich, die Innenstrukturen aus anderen Materialien zu formen, beispielsweise zu stanzen, insbesondere aus Metallfolien. Die Innenstruktur wird nach der Fertigung an einer Folie eines Folienblisters angebracht, vorzugsweise angesiegelt oder direkt auf die Folie gespritzt. Auch eine einstückige Herstellung einer Folie inklusive Innenstruktur ist möglich.

Die Medikamentenmagazine werden in bevorzugten Ausführungsformen derart in einen Inhalator eingebracht, dass eine Innenstruktur direkt in einen Inhalationskanal ausgedrückt wird. Wird das Ausdrücken nach unten ausgeführt, so fällt ein Pulver aufgrund der Schwerkraft aus der geöffneten Medikamentenkammer. In beiden Fällen wird das Pulver aufgrund eines Luftstromes im Inhalationskanal, z. B. durch eine am Inhalator einatmende Person, mitgenommen, wobei im ersten Fall der Luftstrom das Pulver direkt aus der Innenstruktur auslöst. Ein solcher Luftstrom kann auch im zweiten Fall unterstützend wirken.

Die Medikamentenmagazine werden bevorzugt in einem Mehrdosispulverinhalator eingesetzt. Die Anzahl der im Magazin untergebrachten Einzeldosen liegt vorzugsweise in einem Bereich von 1 bis 100 oder bis 200 Einzeldosen, bevorzugt in einem Bereich von 1-60, beispielsweise zwischen 7-180 oder 14-150, z.B. 30-120, 45-100, 30, 90, 60, 120. Für Inhalationsgeräte liegt eine Maximalanzahl von Einzeldosen aus Handlichkeits- und Therapie-Gründen vorzugsweise bei 60.

Typische Mehrdosispulverinhalatoren sind beispielsweise aus US 5,590,645, US 4,627,432, US 6,655,381 oder WO 2005/002654 bekannt. Darin werden unterschiedlichste Medikamentenmagazine, beispielsweise in der Form von Bänder oder als Ringmagazine, fest oder flexibel, mit integrierten oder separaten Medikamententaschen und entsprechend unterschiedlichen Öffnungsmechanismen, wie Anstechen oder Peelen, in verschiedenen Kombinationen, beschrieben.

Die erfindungsgemässen Blister mit Innenstruktur werden bevorzugt in solchen Mehrdosispulverinhalatoren eingesetzt. Die in den genannten Schriften verwendeten und beschriebenen Medikamente und Pulver sind Beispiele für die Art, Zusammensetzung und Pulvergrösse von in den erfindungsgemässen Blistern verwendbaren Pulvern und Medikamenten. Die in den Schriften genannten Inhalatoren sind auch bezüglich Grösse, Anwendung und allgemeinem Aufbau Inhalatoren, wie sie sich - bis auf den eigentlichen Öffnungsmechanismus - für die erfindungsgemässen Medikamentenmagazine eignen.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Besonders bevorzugt sind in diesem Zusammenhang Arzneimittel, die ausgewählt sind aus der Gruppe bestehend aus Anticholinergika, Betamimetika, Steroiden, Phosphodiesterase IV-inhibitoren, LTD4-Antagonisten und EGFR-Kinase-Hemmer, Antiallergika, Derivate von Mutterkornalkaloiden, Triptane, CGRP-Antagonisten, Phosphodiesterase-V-Inhibitoren, sowie Kombinationen aus solchen Wirkstoffen, z.B. Betamimetika plus Anticholinergika oder Betamimetica plus Antiallergika. Bevorzugt werden Anticholinergika-haltige Wirkstoffe eingesetzt, als Monopräparate oder in Form von Kombinationspräparaten.

Im einzelnen seien als Beispiele für die wirksamen Bestandteile oder deren Salze genannt:

Zur Anwendung gelangende Anticholinergika sind bevorzugt ausgewählt aus der Gruppe bestehend aus Tiotropiumbromid, Oxitropiumbromid, Flutropiumbromid, Ipratropiumbromid, Glycopyrroniumsalze, Trospiumchlorid, Tolterodin, 2,2-Diphenylpropionsäuretropenolester-methobromid, 2,2-Diphenylpropionsäurescopinester-methobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxy-fluoren-9-carbonsäuretropenolester -Methobromid, 9-Fluor-fluoren-9-carbonsäuretropenolester -Methobromid, 9-Hydroxy-fluoren-9-carbonsäurescopinester-Methobromid, 9-Fluor-fluoren-9-carbonsäurescopinester Methobromid, 9-Methyl-fluoren-9-carbonsäuretropenolester Methobromid, 9-Methyl-fluoren-9-carbonsäurescopinester Methobromid, Benzilsäurecyclopropyltropinester-Methobromid, 2,2-Diphenylpropionsäurecyclopropyltropinester-Methobromid, 9-Hydroxy-xanthen-9-carbonsäurecyclopropyltropinester-Methobromid, 9-Methyl-fluoren-9-carbonsäurecyclopropyltropinester-Methobromid, 9-Methyl-xanthen-9-carbonsäurecyclopropyltropinester-Methobromid, 9-Hydroxy-fluoren-9-carbonsäurecyclopropyltropinester-Methobromid, 4,4'-Difluorbenzilsäuremethylestercyclopropyltropinester-Methobromid, 9-Hydroxy-xanthen-9-carbonsäuretropenolester-Methobromid, 9-Hydroxy-xanthen-9-carbonsäurescopinester Methobromid, 9-Methyl-xanthen-9-carbonsäuretropenolester -Methobromid, 9-Methyl-xanthen-9-carbonsäurescopinester -Methobromid, 9-Ethyl-xanthen-9-carbonsäuretropenolester Methobromid, 9-Difluormethyl-xanthen-9-carbonsäuretropenolester -Methobromid und 9-Hydroxymethyl-xanthen-9-carbonsäurescopinester -Methobromid, gegebenenfalls in Form ihrer Racemate, Enantiomere oder Diastereomere und gegebenenfalls in Form ihrer Solvate und/oder Hydrate.

Zur Anwendung gelangende Betamimetika sind bevorzugt ausgewählt aus der Gruppe bestehend aus Albuterol, Bambuterol, Bitolterol, Broxaterol, Carbuterol, Clenbuterol, Fenoterol, Formoterol, Hexoprenaline, Ibuterol, Indacaterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenot, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethylphenyl)-ethylamino]-hexyloxy}-butyl)-benzolsulfonamid, 5-[2-(5,6-Diethyl-indan-2-ylamino)-1-hydroxy-ethyl]-8-hydroxy-1*H*-quinolin-2-on, 4-hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2- {4-[3-(4-methoxyphenyl)-1,2,4-triazol-3-yl]-2-methyl-2-butylamino}ethanol , 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol und 1-(4-ethoxycarbonylamino-3-cyano-5-fluorophenyl)-2-(tert.-butylamino)ethanol, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende Steroide sind bevorzugt ausgewählt aus der Gruppe bestehend aus Prednisolon, Prednison, Butixocortpropionat, RPR-106541, Flunisolid, Beclomethason, Triamcinolon, Budesonid, Fluticason, Mometason, Ciclesonid, Rofleponid, ST-126, Dexamethason, 6α,9α-Difluoro-17α-[(2-furanylcarbonyl)oxy]-11β-hydroxy-16α-methyl-3-oxo-androsta-1,4-dien-17β-carbothionsäure (S)-fluoromethylester, 6α,9α-Difluoro-11β-hydroxy-16α-methyl-3-oxo-17α-propionyloxy-androsta-1,4-dien-17β-carbothionsäure (S)-(2-oxo-tetrahydro-furan-3S-yl)ester und Etiprednol-dichloroacetat (BNP-166), gegebenenfalls in Form ihrer Racemate, Enantiomere oder Diastereomere und gegebenenfalls in Form ihrer Salze und Derivate, ihrer Solvate und/oder Hydrate.

Zur Anwendung gelangende PDE IV-Inhibitoren sind bevorzugt ausgewählt aus der Gruppe bestehend aus Enprofyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, CDP840, Bay-198004, D-4418, PD-168787, T-440, T-2585, Arofyllin, Atizoram, V-11294A, Cl-1018, CDC-801, CDC-3052, D-22888, YM-58997, Z-15370, 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(*tert*-butyl)-9*H*-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende LTD4-Antagonisten sind bevorzugt ausgewählt aus der Gruppe bestehend aus Montelukast, 1-(((R)-(3-(2-(6,7-Difluoro-2-quinolinyl)ethenyl)phenyl)-3-(2-(2-hydroxy-2-propyl)phenyl)thio)methylcyclo-propan-essigsäure, 1-(((1(R)-3(3-(2-(2,3-Dichlorothieno[3,2-b]pyridin-5-yI)-(E)-ethenyl)phenyl)-3-(2-(1-hydroxy-1-methylethyl)phenyl)propyl)thio)methyl)cyclo-propanessigsäure, Pranlukast, Zafirlukast, [2-[[2-(4-tert-Butyl-2-thiazolyl)-5-benzofuranyl]oxymethyl]phenyl]essigsäure, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Zur Anwendung gelangende EGFR-Kinase-Hemmer sind bevorzugt ausgewählt aus der Gruppe bestehend aus Cetuximab, Trastuzumab, ABX-EGF, Mab ICR-62, 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluorphenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluorphenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenylethyl)amino]-6- {[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluorphenyl)amino]-6-{1-[(morpholin-4-yl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4- {N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluorphenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methylpiperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluorphenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy} -7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-ammo)-cyclohexan-1-yloxy]-7-methoxy-chinazolm, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluorphenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die Verbindungen gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als Antiallergika: Dinatriumcromoglicat, Nedocromil.

Als Derivate der Mutterkomalkaloide: Dihydroergotamin, Ergotamin.

Für die Inhalation kommen Arzneimittel, Arzneimittelforrnulierungen und - mischungen mit den o.g. Wirkstoffen in Betracht, sowie deren Salze, Ester sowie die Kombination dieser Wirkstoffe, Salze und Ester.

Im Folgenden wird der Erfindungsgegenstand anhand von bevorzugten Ausführungsbeispielen, welche in den beiliegenden Zeichnungen dargestellt sind, näher erläutert. Es zeigen jeweils schematisch:
- Figur 1a-1e: einen Folienblister mit Innenstruktur,
- Figur 2a-2c: weitere Innenstrukturen,
- Figur 3a-3c: einen Folienblister mit symmetrischer Innenstruktur,
- Figur 4a-c: einen Folienblister mit asymmetrischer Innenstruktur,
- Figur 5a-5c: eine Innenstruktur mit Luftstrom durch das Innere der Innenstruktur,
- Figur 6a-6d: eine weitere Innenstruktur mit Luftstrom durch die Innenstruktur,
- Figur 7a, 7b: eine Innenstruktur als Zentrier- und Stechhilfe für Saugnadeln,
- Figur 8a, 8b: eine weitere Innenstruktur als Zentrier- und Stechhilfe,
- Figur 9a, 9b: eine Innenstruktur mit zweiseitigen Stechpunkten oder Schneidkanten,
- Figur 10a, 10b: eine Innenstruktur mit seitlichen Luftzufuhrkanälen.

Grundsätzlich sind in den Figuren gleiche Teile mit gleichen Bezugszeichen versehen. Die beispielhaft beschriebenen Medikamentenmagazine in der Form eines Folienblisters weisen jeweils eine untere und eine obere Folie auf, zwischen welchen eine Innenstruktur eingebracht ist. In den Figuren ist eine erste untere Folie diejenige, in welche die Innenstruktur eingebracht ist und welche dazu in der Regel eine Vertiefung aufweist, bzw. dadurch eine Vertiefung eingeformt wird. Die zweite Folie wird entsprechend als Siegelfolie bezeichnet, da sie in einem Herstellungsprozess zur Versiegelung über einer gefüllten Innenstruktur bzw. Medikamentenkammer angebracht wird. Je nach Material und Herstellungsprozess des Magazins sind die beiden Folien symmetrisch angeordnet. Die Medikamentenkammern sind bevorzugt Teile eines Medikamentenmagazins mit einer Vielzahl von nebeneinander angeordneten Medikamentenkammern mit Medikamenteneinzeldosen.

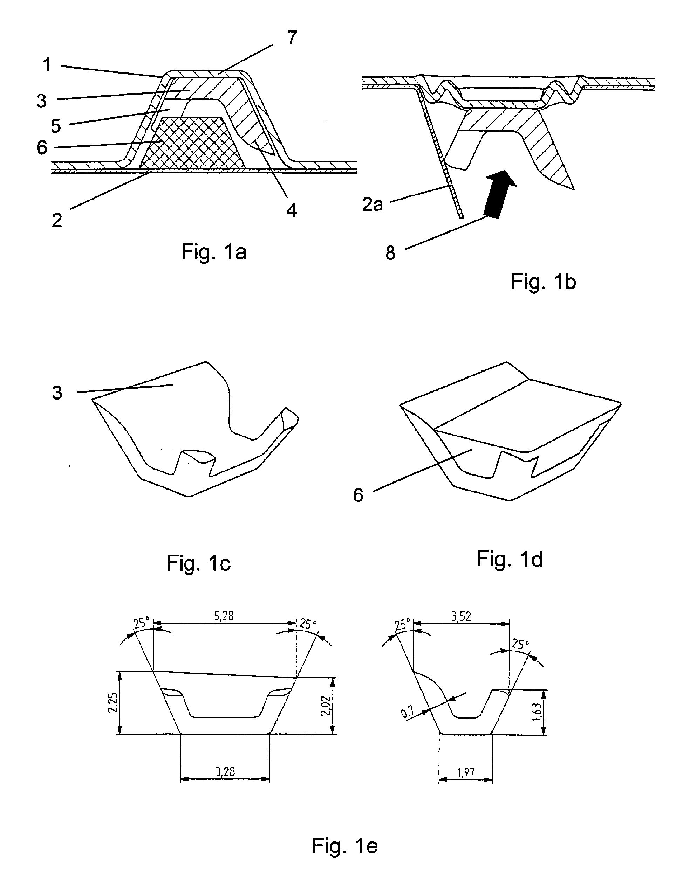

**Figur 1a** zeigt einen Schnitt durch eine Medikamentenkammer in einem Folienblister. Der Blister weist eine erste Folie 1, eine Innenstruktur 3 und eine mit der ersten Folie 1 dicht verschlossene Siegelfolie 2 auf. Die Innenstruktur 3 weist eine Seitenwand mit einer Schneidkante 4 auf, sowie auf der der Schneidkante gegenüberliegenden Seite zwei Beine 5 **(Figur 1c).** Die Beine 5 sind gegenüber der Schneidkante 4 zurückversetzt und unterstützen den Öffnungsvorgang der Medikamentenkammer. Wirkt eine Kraft auf die Rückseite des Blisters, so wird zuerst die Schneidkante 4 gegen die Siegelfolie 2 gedrückt, wobei diese aufgeschnitten wird. Anschliessend drücken die Beine 5 den zuvor von der Schneidkante geöffneten Folienlappen 2a zur Seite, wie in **Figur 1b** gezeigt. Das im Blister vorhandene Pulver 6 kann nun herausfallen und/oder durch einen Luftstrom 8, in der Figur mit einem Pfeil eingezeichnet, dem Blister entnommen werden.

Die Innenstruktur ist an ihrer Rückseite mit der ersten Folie versiegelt. Dies hat den Vorteil, dass die Innenstruktur auch bei vollständig geöffnetem Blister nicht herabfallen und/oder sich als separates Teil verselbständigen kann. Mit dieser Art eines Blisters fällt zudem kein Material an, das separat gesammelt werden muss. Die Innenstruktur kann auch zusätzlich auf den Längsseiten der Innenstruktur versiegelt sein, so dass kein Pulver 6 hinter diese Seitenwandabschnitte gelangen kann.

Die Innenstruktur 3 weist auch eine gewisse Stabilität auf, so dass das in ihm befindliche Pulver 6 vor mechanischen Einflüssen von aussen, insbesondere vor einer Kompaktierung durch Druck, teilweise oder gänzlich geschützt ist.

In den **Figur 1d und 1e** ist eine Schrägansicht und zwei Seitenansichten inklusive einer beispielhaften Vermassung der Innenstruktur für ein beispielhaftes Pulvervolumen von ca. 10mm³ dargestellt. In den Figuren ist zudem zu sehen, dass die Schneidkante leicht geneigt ist, so dass die Siegelfolie zu Beginn der Öffnung von einer Ecke der Schneidkante durchstochen wird und erst anschliessend auf der gesamten Breite der Innenstruktur, entlang der Schneidkante aufgeschnitten wird. Es wäre auch möglich, die Schneidkante durch einen Stechpunkt und eine Querstrebe zu ersetzen. Dabei würde die Folie ebenfalls an einer Ecke aufgestochen, die restliche Breite der Folie würde dabei aber nicht kontrolliert aufgeschnitten, sondern gerissen.

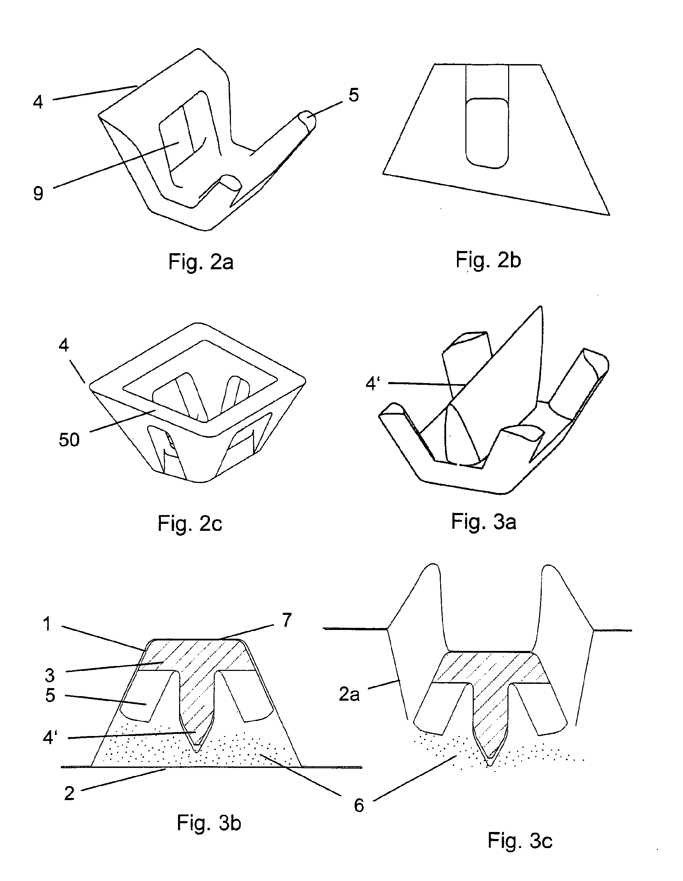

Die **Figuren 2a, 2b und 2c** zeigen weitere Beispiele von Innenstrukturen mit einer seitlichen Schneidkante und einem im wesentlichen rechteckigen oder quadratischen Grundriss. Die Figur 2a weist zudem zwei Aufstossbeine 5 auf. Die Seitenwand, welche die Schneidkante 4 beinhaltet weist eine Öffnung und einen dadurch entstehenden Hohlraum 9 auf. Diese Öffnung bewirkt, dass die Menge von Pulver, welche möglicherweise hinter die Seitenwand gelangt möglichst kein ist: Pulver kann durch die Öffnung wieder in das Innenvolumen der Innenstruktur gelangen und wird nicht zwischen Seitenwand und Folie eingeklemmt.

In der Innenstruktur gemäss den Figuren 2b und 2c sind die Beine als Eckpfeiler ausgestaltet, welche über Querstreben 50 miteinander und mit der Schneidkante verbunden sind. Die Querstreben ermöglichen ein kontrollierteres Schneiden oder Aufdrücken der Siegelfolie, je nach dem ob die Querstreben zusätzlich als Schneidkanten ausgebildet sind. Die Innenstruktur weist wiederum an unterschiedlichen Ecken unterschiedlich Höhen auf, so dass ein Öffnen der Siegelfolie an einer Ecke beginnt und kontrolliert über die nachfolgenden Kanten bzw. Querstreben verläuft.

In den **Figuren 3a bis 3c** ist eine symmetrische Innenstruktur 3 mit mittig angeordneter Schneidkante 4' und entsprechendem Öffnungsvorgang eines Blisters dargestellt. Die Innenstruktur, hier der Einfachheit halber mit im wesentlichen rechteckigem oder quadratischem Grundriss gezeigt, weist über ihre gesamte Breite eine Schneidkante auf. An allen vier Ecken sind zudem vier gegenüber der Schneidkante leicht zurückversetzte Beine 5 als zusätzliche Öffnungshilfen angebracht.

Das im Blister befindliche Pulver 6 ist durch das durch die Schneidkante und Beine aufgespannte Volumen vor Druck geschützt. Der Blister ist wiederum an seiner Rückseite mit der einen Folie 1 versiegelt, während die Siegelfolie 2 den Blister geschlossen hält. Wird der Blister durch Krafteinwirkung auf seine Hinterseite geöffnet, so schneidet die Schneidkante 4' die Siegelfolie 2 durch, wobei mit Hilfe der Beine 5 zwei Folienlappen 2a entstehen. Diese Folienlappen 2a sind kürzer als bei einer seitlichen Schneidkante und verringern damit das Risiko sich zu lösen, hängenzubleiben oder beim Pulverentnahmevorgang im Weg zu sein. In den Schnittzeichnungen der Figuren 3b und 3c ist wiederum die einseitig erhöhte Schneidkante zu erkennen. Aufgrund der Siegelung 7 an der Rückseite der Innenstruktur bleibt diese an der ersten Folie haften.

Eine symmetrische Form der Innenstruktur weist den Vorteil der einfacheren Herstellung und Handhabung auf.

Ein bevorzugtes Rückhaltemittel ist eine Ansiegelung der Innenstruktur an eine Folie. Die Innenstruktur kann jedoch auch anders an einer Folie befestigt werden, beispielsweise mechanisch, z. B. in der Form einer Niete oder eines Druckknopfes. Dabei weist die Innenstruktur an ihrer hinteren an einer Folie anzubringenden Seite eine Struktur auf, welche mit einer korrespondierenden Form einer Aussenstruktur klemmbar ist. Die eine Folie eines Blisters wird dabei zwischen der Innenstruktur und der Aussenstruktur eingeklemmt. Solche Aussenstrukturen sind vorzugsweise integraler Bestandteil eines Medikamentenmagazins und können je nach Ausgestaltung auch noch weitere Funktionen innehaben: Ist ein Medikamentenmagazin bandförmig ausgestaltet, können Aussenstrukturen beispielsweise als Führungen für das Band benutzt werden; Sie können direkt für einen Öffnungsmechanismus verwendet werden, in dem über die Aussenstruktur Druck auf das Magazin und damit auf die Innenstruktur ausgeübt wird; Sie können als Index verwendet werden zum Anzeigen einer Magazinposition etc..

Solche mechanische, vorzugsweise klemmende Rückhaltemittel sind dann vorteilhaft, wenn eine Siegelung nicht möglich oder erwünscht ist oder wenn weitere Funktionen, wie beispielsweise oben genannt, in ein Medikamentenmagazin integriert werden sollen.

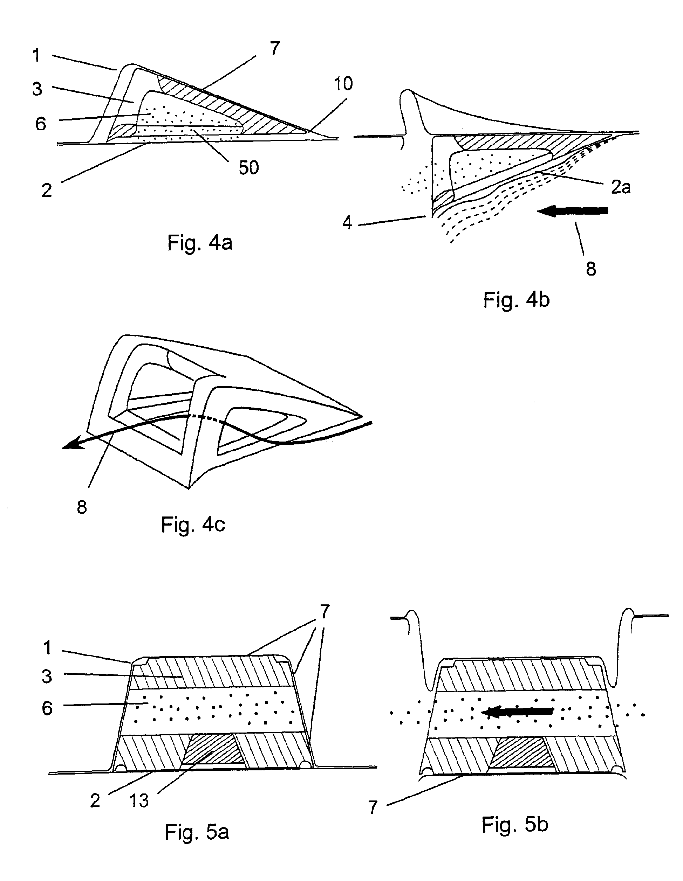

In den **Figuren 4a bis 4c** ist ein Blister mit Innenstruktur gezeigt, welche einerseits asymmetrisch ist und nebst einer Siegelung 7 an ihrer Rückseite auch eine andere Art eines Rückhaltemechanismus erlaubt. Die Innenstruktur in dieser Ausführungsform hat im wesentlichen den seitlichen Querschnitt eines rechtwinkligen Dreiecks, welcher Querschnitt das Öffnen des Blisters durch ein Drücken auf die Hinterseite des Blisters ermöglicht. Aufgrund der rampenartigen Dreieckform der Rückseite der Innenstruktur ist ein Drücken parallel zu und entlang des Folienblisters (welcher in Zeichenebene von rechts nach links verläuft) möglich. Dies kann sehr einfach durch ein Drüberrollen, beispielsweise einer mit Druck beaufschlagten Rolle, über die Hinterseite des Blisters ausgeführt werden. Es ist auch möglich, dass ein Blisterband beim Weitertransportieren an einem z. B. rampenförmigen Element 'ausgedrückt' wird (dabei würde sich das Band entsprechend von links nach rechts). Damit kann eine Vorrichtung kompakter gestaltet werden, da beispielsweise kein Hub eines Krafteinwirkungselements nötig ist.

Durch die Kraft, die auf die schräge Rückseite des Blisters einwirkt, schneidet eine auf der Vorderseite der Innenstruktur 3 befindliche Schneidkante 4 in die Siegelfolie 2 und gräbt sich, ähnlich einer Baggerschaufel, weiter durch diese. Querstreben 50, die von der Schneidkante im wesentlichen parallel zur Siegelfolie 2 verlaufen und welche vorzugsweise auch als Schneidkanten ausgestaltet sind, schneiden die Siegelfolie auf ihrer gesamten Länge und führen zu einem länglichen Folienlappen 2a. Ist die Innenstruktur ganz aus dem Blister bzw. der Medikamentenkammer ausgedrückt, fällt das in der Innenstruktur befindliche Pulver zumindest teilweise aufgrund der Schwerkraft in einen Inhalationsraum (nicht dargestellt). Es ist auch möglich, dass das Pulver gegebenenfalls zusätzlich durch einen im Inhalationsraum wirkenden Luftstrom 8, welcher in den Figuren 4b und 4c durch einen Pfeil eingezeichnet ist, aus der Innenstruktur gelöst wird. Der Folienlappen 2a kann dabei eine unterstützende Funktion beim Auslösen des Pulvers haben, indem der Lappen 2a durch den Luftstrom 8 gegen die Unterseite der Innenstruktur vibriert, und dabei ein Ausklopfen des Pulvers bewirkt. Eine Bewegung des Folienlappens 2a ist in der Figur gestrichelt eingezeichnet. Seitliche und eine vordere Öffnung in der Innenstruktur ermöglichen einen Luftstrom 8 durch die Innenstruktur, wie in Figur 4c eingezeichnet. Dieser sorgt für ein vollständige Auslösen der Pulvers und eine Verwirbelung für eine bessere Loslösung eines Wirkstoffes von einem Trägermaterial (Dispersion).

Um zu Verhindern, dass die Innenstruktur aus dem Blister fällt, kann anstelle oder zusätzlich zur Siegelung 7 an der Rückseite der Innenstruktur an der ersten Folie auch beispielsweise eine Art Scharnier 10 durch Folie(n) und Innenstruktur gebildet sein. Dabei wird die Innenstruktur 3 mit einer Drehbewegung aus dem Blister gedrückt. Zur Bildung eines solchen Scharniers 10 könnte beispielsweise diejenige Kante der Innenstruktur, an welcher Hypothenuse und Ankathete des Dreiecks zusammentreffen, derart verlängert sein, dass die Kante zwischen den beiden Folien festgemacht, z. B. eingeklebt, eingeschweisst oder eingesiegelt ist.

Die in den Figuren 4a-4c gezeigte Innenstruktur wäre auch geeignet, um aus einer Metallfolie geformt, z. B. gestanzt und gefaltet, zu werden.

Nicht nur das Ausdrücken der Innenstruktur kann in dieser Ausführungsform sehr kompakt ausgeführt sein. Auch das Rückführen der Innenstruktur zurück auf eine Ebene in unausgedrücktem Zustand des Blisters, beispielsweise zwecks Weitertransport eines Blisterbandes, kann durch ein Anpressen der Innenstruktur an ein z.B. rampenartiges Element erfolgen. Dadurch können während einem Weitertransport des Blisterbandes mehrere Aktionen gleichzeitig erfolgen: der Transport und das Rückführen der Innenstruktur in seine ursprüngliche Position, gegebenenfalls auch das Ausdrücken eines neuen Blisters.

In dieser Figur entspricht eine typische Bewegungsrichtung eines Blisterbandes auch einer, gegebenenfalls entgegengesetzten, Entnahmerichtung. In bevorzugten Ausführungsformen eines Inhalators, wird eine Entnahme aus Platzgründen im wesentlichen senkrecht zu einer Bewegungsrichtung eines Medikamentenmagazins angeordnet sein.

In den **Figuren 5a** und **5b** ist ein Schnitt durch einen Blister 1 mit einer schachtelförmigen Innenstruktur 3 gezeigt. Die Schachtel weist in der Mitte einer Vorderseite eine Einfüllbohrung 13 zum Einfüllen eines Pulvers 6 auf, welche nach dem Füllen mit einem Stopfen verschlossen wird. Die Innenstruktur ist derart zwischen zwei Folien eingebracht, dass eine obere Siegelfolie 2 die Einfiillbohrung verschliesst, so dass gegebenenfalls auf einen Stopfen verzichtet werden kann. Das eingefüllte Pulver 6 befindet sich nun vorzugsweise vollständig in einem Kanal im Inneren der Innenstruktur. Dieser Kanal verläuft längs durch die Innenstruktur und ist mit der Einfüllbohrung 13 verbunden.

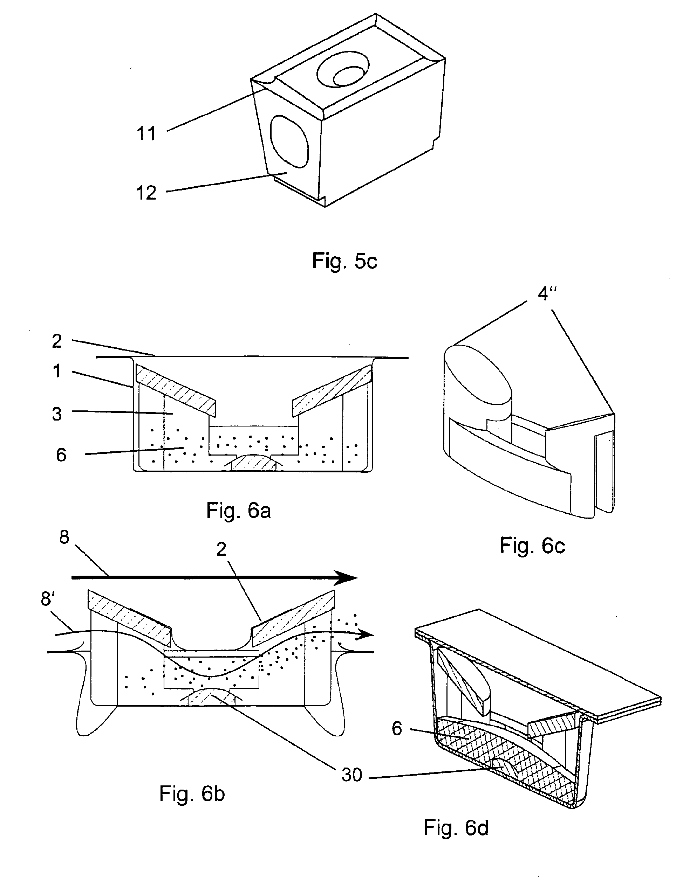

Schneidkanten 11, welche auf der Vorderseite und rings um die Innenstruktur verlaufen, siehe Figur 5c, schneiden ein Loch in die Siegelfolie 2, welche der Fläche der Innenstruktur entspricht. Aufgrund einer Siegelung 7 auf der Vorderseite der Innenstruktur bleibt das ausgeschnittene Folienstück an der Innenstruktur haften. Der geöffnete Blister wird nun vorzugsweise derart positioniert, dass die Innenstruktur sich in einem Inhalationskanal befindet. Ein Luftstrom im Inhalationskanal, mit einem Pfeil eingezeichnet, führt durch den Kanal in der Innenstruktur und nimmt dabei das darin befindliche Pulver 6 mit. Durch die Siegelfolie 2 auf der Innenstruktur 3 und/oder durch den Stopfen wird verhindert, dass Pulver an einer anderen Stelle als durch die Öffnungen des Kanals, den Austrittsöffnungen, austreten kann.

Eine weitere Siegelung 7 ist wiederum zwischen erster Folie und Rückseite der Innenstruktur vorhanden, um ein Abfallen der Innenstruktur von der Folien zu verhindern. Eine Siegelung 7 der ersten Folie 1 kann aber auch auf denjenigen Seiten der Innenstruktur angebracht sein, welche die Austrittöffnungen aufweisen. Dadurch gerät kein Pulver ausserhalb der Innenstruktur in die Medikamentenkammer. Die seitlichen Siegelungen werden beim Öffnungsvorgang der Blisters von der Innenstruktur abgelöst, so dass die Austrittsöffnungen freigegeben werden.

In der beschriebenen Ausführungsform der Innenstruktur ist ein Pulver vollständig vor einem Zusammendrücken, bei der Lagerung, aber auch bei einem Öffnen des Blisters geschützt. Zudem erlaubt eine von einer oder mehreren Austrittsöffnungen unabhängige Einfüllöffnung mehr Spielraum in Bezug auf eine Entnahme bzw. eine Dosierung eines Medikaments. Diese werden wesentlich durch die Austrittsöffnungen, allgemein durch den Entnahmewiderstand aus der Innenstruktur, bestimmt. Eine Einfüllöffnung ist jedoch vorzugsweise möglichst gross, so dass ein Einfüllen schnell und einfach vorgenommen werden kann. Zudem sollte eine Einfüllöffnung auf einer oberen Seite eines Blisters sein, während Entnahmeöffnungen, wie im vorliegenden Fall, je nach Inhalatoranordnung seitlich angeordnet sein können.

Eine weitere Ausführungsform, welche eine von Austrittsöffnungen unabhängige Einfüllöffnung in einer Innenstruktur aufweist, ist in den F**iguren 6a bis 6d** gezeigt. Ein Pulver 6 wird wiederum durch eine der Siegelfolie 2 gegenüberliegende Einfüllöffnung eingefüllt. Rückwärtige und seitliche Öffnungen sind mit einer ersten Folie 1 verschlossen, wobei die seitlichen Öffnungen beim Öffnungsvorgang von der Folie 1 abgelöst werden und einen Durchgang durch die Innenstruktur zum Durchströmen von Luft freigeben. In der Figur 6b ist ein über die Struktur führender Luftstrom 8, sowie ein durch die Innenstruktur führender abgelenkter Luftstrom 8' eingezeichnet. In der Figur 6c sind zwei bogenförmige Schneidkanten 4" zu sehen, welche zwei voneinander beabstandete Öffnungen in die Siegelfolie 2 schneiden. Bei einem Herausdrücken der Innenstruktur aus dem Blister wird die Siegelfolie in einem Zwischenbereich zwischen den Schneidkanten an die Innenstruktur gedrückt (siehe Fig. 6b), so dass damit einerseits die Einfüllöffnung verschlossen wird und andererseits die Innenstruktur im Blister gehalten wird, ohne dass eine Rückseite versiegelt sein muss. Die Innenstruktur weist im Pulverbereich ein Verwirbelungselement 30 auf, welches für zusätzliche Turbulenzen bei der Entnahme des Pulvers sorgt und dadurch eine Deagglomeration des Pulvers unterstützt.

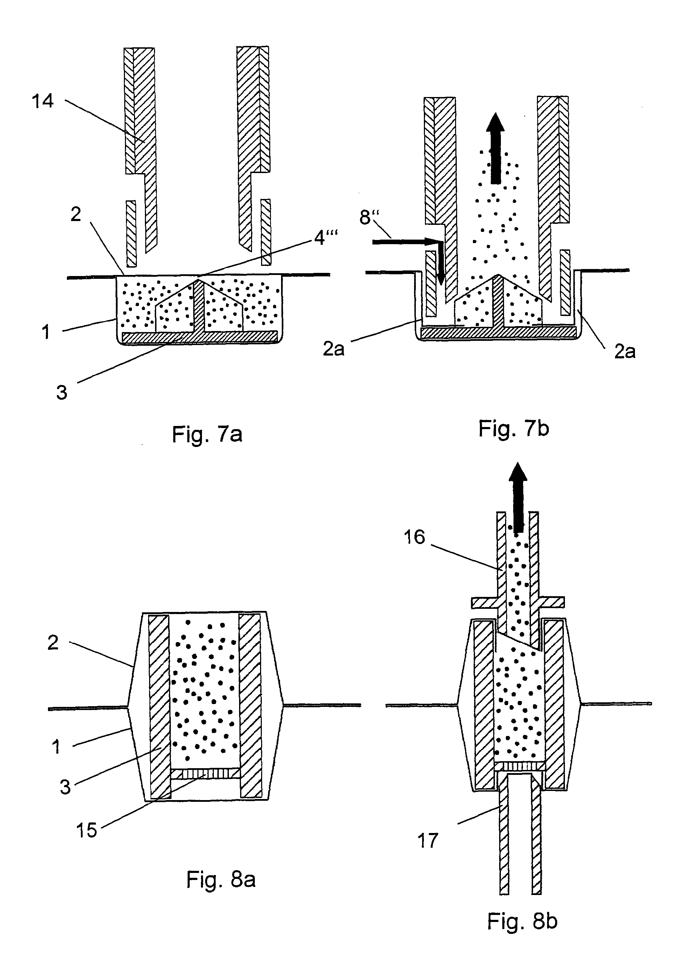

In den **Figuren 7a, 7b, 8a und 8b** sind Innenstrukturen dargestellt, welche nebst einer Stabilisierungsfunktion, auch Stech- und Zentrierhilfen für von aussen in den Blister 1 einzuführende Saugnadeln, Kanülen, Luftzufuhrnadeln etc. sind.

In der Figur 7a und 7b ist die Innenstruktur 3 im wesentlichen symmetrisch und weist einen zentral angebrachten Stechpunkt oder eine Schneidkante 4"' auf. Die Innenstruktur 3 ist in Kombination mit einer Doppelkanüle 14 gezeigt. Die doppelwandige Kanüle ist so ausgeführt, dass durch ihr Inneres gesaugt werden kann (dicker Pfeil), während durch den Aussenteil die Luftzufuhr 8" garantiert wird. Die Innenstruktur besteht aus einer Platte, welche in etwa denselben Durchmesser aufweist wie die Kanüle 14. Im Zentrum der Platte sind zur Mitte hin konisch zulaufende Wände aufgebracht, welche als Führung bzw. Anstosspunkte für die einzuführende Kanüle dienen. Dazu weisen die Wände einen leicht grösseren Durchmesser wie der Innendurchmesser der Kanüle auf.

An die erste Folie 1, welche vorgeformt sein kann, wird die Innenstruktur angespritzt, angesiegelt oder auf andere Art eingebracht. Vorzugsweise wird beim Anbringen der Innenstruktur eine entsprechende Vertiefung in die Folie 1 geformt. Anschliessen wird die noch offene Medikamentenkammer gefüllt und mit einer Siegelfolie 2 versiegelt. Beim Anstechen mit der Kanüle 14 wird die Siegelfolie aufgrund der auf sie einwirkenden Kraft in der Mitte des Blisters durch die Innenstruktur angestochen und aufgerissen bzw. -geschnitten. Die freien seitlichen Folienlappen werden durch die Doppelkanüle zur Seite, in die Medikamentenkammer und auf die Platte der Innenstruktur hinuntergedrückt. Ein Luftstrom 8' kann nun von aussen durch die Kanüle, durch die Innenstruktur bzw. an der Innenstruktur vorbei, im Inneren entlang der Kanüle in Saugrichtung nachgeführt werden, wobei dadurch eine im wesentlichen vollständige Entnahme des Pulver möglich ist.

Die Innenstruktur 3 in den Figuren 8a und 8b weist selber keine Stechpunkte oder Schneidkanten auf. Die Innenstruktur besteht im wesentlichen aus einem Rohr oder auch zwei gleich grossen parallelen Wänden, welche in einem unteren Teil über einen Filter 15 verbunden sind. Die Innenstruktur ist vorzugsweise ein Kunststoffteil und der Filter 15 ein integraler Bestandteil davon, welcher im wesentlichen eine mit Filteröffnungen versehene dünne Kunststoffwand ist. Der Filter verhindert das Austreten des im Blister befindlichen Pulvers in ein Gerät bzw. das Eintreten von unerwünschten Kleinteilen in die Kammer durch eine Luftzufuhrnadel 17 (siehe unten).

Die Innenstruktur ist zur Verwendung mit einer Saug- 16 und einer Luftzufuhrnadel 17 ausgelegt. Dazu werden die beiden Folien von oben und unten angestochen, wobei die Saug- und Luftzufuhrnadel eine entsprechende Schneidkante bzw. Stechpunkte aufweist. Der Durchmesser des Rohres ist dabei so bemessen bzw. die beiden Wände der Innenstruktur sind soweit voneinander beabstandet, dass die Nadeln 16, 17 dazwischen eingeführt werden können. Beim Aufschneiden der Folien 1, 2 werden die dabei entstehenden Folienlappen zur Seite zwischen Nadel und Folie bzw. Wand gedrückt, wo sie Nadeln und Medikamentenkammer gegeneinander abdichten.

Während die Luftzufuhrnadel 17 am Filter ansteht, weist die Saugnadel 16 vorzugsweise einen Haltemechanismus auf, beispielsweise einen ringförmigen Rand um die Saugnadel 16, welche an den Wänden der Innenstruktur ansteht und ein definiertes Eindringen der Saugnadel in die Innenstruktur ermöglicht.

Die Innenstruktur könnte anstelle der zwei Wände oder des Rohres auch ein Rechteckzylinder sein, so dass ein in seinem Inneren befindliches Medikament im wesentlichen vollständig eingeschlossen ist. Runde Formen der Innenstruktur werden insofern bevorzugt, da ihre Herstellung, Zentrierung einer Nadel oder ihre Ausrichtung in Bezug auf die Folie vereinfacht sind. Obere und untere Folie können auf der Innenstruktur angesiegelt sein.

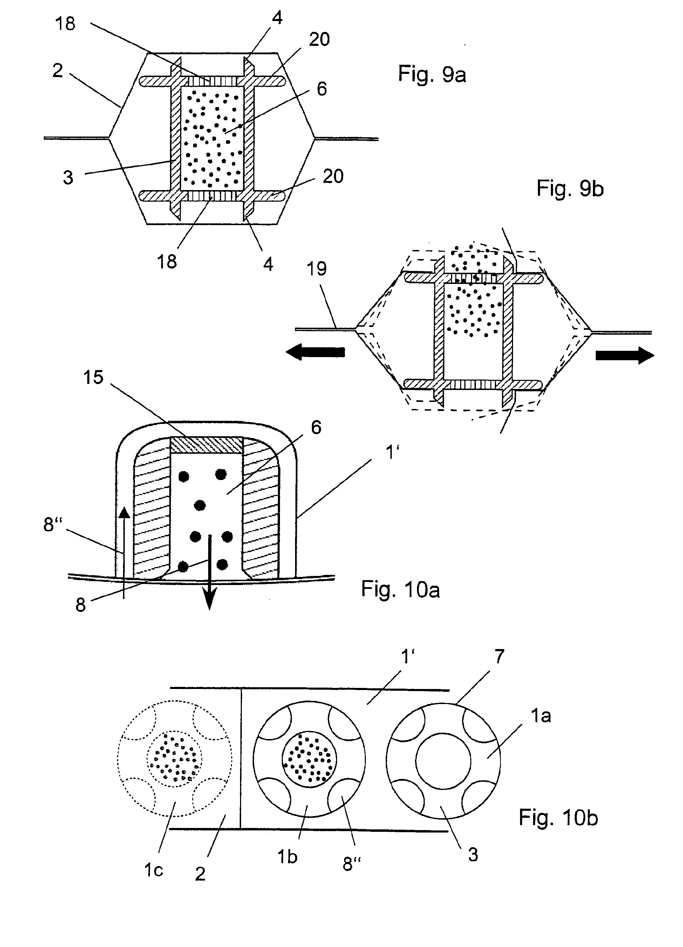

In den **Figuren 9a und 9b** ist eine runde oder eckige käfigartige Innenstruktur 3 gezeigt, welche nicht an einer Folie angesiegelt wird. Als Rückhaltemechanismus, damit die Innenstruktur auch nach dem Öffnen des Blisters nicht heraus- oder abfällt, weist diese ausserhalb der eigentlichen, das Pulvervolumen umfassenden, Innenstruktur, Vorsprünge 20 auf. Das Innenvolumen wird wiederum durch einen runden Zylinder, zwei parallele Wände oder einen rechteckigen Zylinder aufgespannt. Die Wände bzw. Zylinder weisen an ihren Enden Stechpunkte oder Schneidkanten 4 auf, welche die obere und untere Folie 2 durchtrennen. Zwischen den Wänden bzw. im Inneren der Zylinder sind, vorzugsweise integral, Siebe 18 geformt, welche gröbere Partikel im Innenvolumen zurückhalten.

Die Vorsprünge sind ausserhalb der oberen und unteren Schneidkanten 4 angebracht und als Verlängerungen der Siebe 18 gestaltet.

Figur 9a zeigt einen Blister in ungeöffnetem, Figur 9b in geöffnetem Zustand. Ein Öffnen dieses Blister kann durch ein seitliches Ziehen in Pfeilrichtung an den Laschen 19 geschehen. Verschiedene Stadien des Öffnungsvorganges sind in Figur 9b durch gestrichelte Folienpositionen angedeutet.

In den **Figur 10a und 10b** ist eine Innenstruktur 3 mit mehreren Luftzufuhrkanälen gezeigt. Ein Pulver 6 befindet sich im zylindrischen Innenvolumen der im wesentlichen zylinderförmigen Innenstruktur. Der Innenzylinder ist auf einer Seite mit einem Filter 15 versehen, so dass keine Fremdkörper über die Luftzufuhr 8" in ein Pulver und damit in einen Inhalationskanal gelangen. In Figur 10b ist eine Aufsicht auf ein Blisterband 1' mit drei Medikamentenkammern in verschiedenen Gebrauchsstadien dargestellt. Eine Medikamentenkammer oder Blister 1a ist leer, wobei sich die Innenstruktur weiterhin im Blisterband 1' befindet, beispielsweise durch eine Siegelung 7 von Seitenwänden der Innenstruktur mit dem Blisterband 1'. Ein zweiter Blister 1b ist geöffnet und zur Entnahme des Pulvers bereit. Ein dritter Blister 1c ist noch mit einer Siegelfolie 2 verschlossen.

Die Kanäle für die Luftzufuhr können auch filterseitig in die Innenstruktur hineinversetzt sein, derart, dass auch ein Andrücken einer hinteren Folie die Kanäle nicht beinträchtigt oder die Luftzufuhr unterbinden kann.

Das Öffnen der Medikamentenkammer kann durch abziehen, abschaben oder auch aufstechen der Folie im Bereich der Öffnungen geschehen.

Eine in dieser Figur beschriebene Innenstruktur stabilisiert eine Medikamentenkammer und schützt das in ihr befindliche Pulver vor einem Zusammendrücken. Die Entnahme des Pulvers erfolgt jedoch ohne ein Ausdrücken der Innenstruktur aus der Folie. Das Blisterband 1' kann deshalb auch eine sehr dünne, weiche Folie sein, da die Medikamentenkammer durch die Innenstruktur stabilisiert, insbesondere auch die Luftkanäle durch die Innenstruktur freigehalten werden. Umgekehrt kann auch eine sehr stabile und gegebenenfalls für eine Ausdrücken ungeeignete Folie verwendet werden. Dies würde beispielsweise ein Formen dieser Folie ermöglichen, welche Form aufgrund ihrer Stabilität ein Offenhalten der Luftzufuhrkanäle unterstützen kann.

## Patentansprüche

1. Medikamentenmagazin mit mindestens einer Medikamentenkammer zur Verwendung in einer Inhalationsvorrichtung, wobei das Magazin aus zwei aneinander angebrachten Folienbahnen gebildet ist, und zwischen den Folienbahnen die mindestens eine Medikamentenkammer ausgebildet ist **dadurch gekennzeichnet, dass** die Medikamentenkammer eine Innenstruktur aufweist, welche Innenstruktur (3) an einer Vorderseite ein Mittel zum Öffnen der einen Folienbahn aufweist und dass ein Rückhaltemittel vorhanden ist, welches die Innenstruktur nach einem Öffnen der Medikamentenkammer am Medikamentenmagazin hält.

2. Medikamentenmagazin mit mindestens einer Medikamentenkammer zur Verwendung in einem Pulverinhalator, wobei das Magazin aus zwei aneinander angebrachten Folienbahnen gebildet ist, und zwischen den Folienbahnen die mindestens eine Medikamentenkammer ausgebildet ist **dadurch gekennzeichnet, dass** die Medikamentenkammer eine Innenstruktur (3) aufweist, welche Innenstruktur in ihrem Inneren ein Volumen zur Aufnahme eines pulverförmigen Medikaments aufweist und dieses Innenvolumen gegenüber mechanischen Einflüssen von Aussen stabilisiert.

3. Medikamentenmagazin nach Anspruch 1 oder 2, wobei die Innenstruktur (3) an einer Rückseite mit einer Folienbahn verbunden ist.

4. Medikamentenmagazin nach einen der Ansprüche 1-3, wobei die Innenstruktur (3) ein einstückiges Spritzgusselement ist.

5. Medikamentenmagazin nach einem der Ansprüche 1, 3 oder 4, wobei das Rückhaltemittel durch ein Anstossen der Innenstruktur (3) an nicht geöffneten Bereichen einer Medikamentenkammer gebildet wird.

6. Medikamentenmagazin nach Anspruch 5, wobei die Innenstruktur (3) seitliche Vorsprünge (20) aufweist, zum Eingreifen in Randbereiche der geöffneten Medikamentenkammer.

7. Medikamentenmagazin nach einem der Ansprüche 1,3 oder 4 wobei das Rückhaltemittel in der Form eines Scharniers (10) zwischen Innenstruktur (3) und mindesten einer Folie (1,2) ausgebildet ist.

8. Medikamentenmagazin nach einem der Ansprüche 1, 3 - 7, wobei das Mittel zum Öffnen mindestens ein Stechpunkt oder mindestens eine Schneidkante (4,4',4",4"') ist.

9. Medikamentenmagazin nach Anspruch 8, wobei die Innenstruktur (3) an gegenüberliegenden Seiten mindestens einen Steckpunkt oder Schneidkante aufweist (4,4'), zum Öffnen beider Folienbahnen.

10. Medikamentenmagazin nach Anspruch 8 oder 9, wobei eine Schneidkante (4,4') im wesentlichen über eine gesamte Breite einer Medikamentenkammer verläuft.

11. Medikamentenmagazin nach einem der vorhergehenden Ansprüche, wobei die Innenstruktur (3) mindestens einen vorstehenden Stechpunkt oder Schneidkante (4,4',4",4"') und vorstehende, in Bezug auf den Stechpunkt oder die Schneidkante zurückversetzte, Öffnungshilfen aufweist.

12. Medikamentenmagazin nach Anspruch 11, wobei die Öffnungshilfen als von der mindestens einen Schneidkante (4,4',4",4"') beabstandet angebrachte einzelne Beine (5) und/oder als Querstreben (50) zwischen dem mindestens einen Stechpunkt oder der Schneidkante und weiteren Bereichen der Innenstruktur (3) ausgebildet sind.

13. Medikamentenmagazin nach Anspruch 11 oder 12, wobei die Innenstruktur (3) symmetrisch ausgebildet ist und eine mittig angeordnete Schneidkante (4',4'''), sowie an vier Eckbereichen der Innenstruktur jeweils ein Bein (3) zum Aufklappen von durch die Schneidkante aufgerissenen Folienbereichen aufweist.

14. Medikamentenmagazin nach Anspruch 11 oder 12, wobei die Innenstruktur (3) asymmetrisch aufgebaut ist und die Schneidkante (4) durch eine Seitenwand der Innenstruktur gebildet wird bzw. der Stechpunkt Teil einer Seitenwand der Innenstruktur ist.

15. Medikamentenmagazin nach einem der Ansprüche 11, 12 oder 14, wobei von der die Schneidkante (4) oder den Stechpunkt aufweisenden Seitenwand unter einem Neigungswinkel zwei parallele Querstreben (50) zu der der Seitenwand gegenüberliegenden Seite der Innenstruktur (3) verlaufen.

16. Medikamentenmagazin nach einem der Ansprüche 12-14, wobei die Querstreben (50) als Schneidkanten (4) ausgebildet sind.

17. Medikamentenmagazin nach einem der Ansprüche 14-16, wobei die Querstreben (50) an ihrem einen Ende mit der Seitenwand und mit ihrem anderen Ende mit einer Rückwand der Innenstruktur (3) oder mit an der Rückwand angebrachten Enden von Beinen (5) verbunden sind.

18. Medikamentenmagazin nach einem der Ansprüche 12-17, wobei durch Querstreben (50) und Beine (5) bzw. Rückwand eine oder mehrere Öffnungen in der Innenstruktur (3) gebildet werden, zum Austreten eines sich im Innenbereich der Innenstruktur befindlichen Medikaments.

19. Medikamentenmagazin nach einem der Ansprüche 1-4, wobei die Innenstruktur (3) eine Einfüllöffnung zum Einfüllen eines Medikaments und eine von der Einfüllöffnung unterschiedliche Austrittsöffnung aufweist.

20. Medikamentenmagazin nach Anspruch 19, wobei die Innenstruktur (3) eine Durchströmöffnung aufweist, zum Durchströmen der Innenstruktur durch die Durchströmöffnung und die Austrittsöffnung hindurch, nach einem Öffnen der Medikamentenkammer.

21. Medikamentenmagazin nach Anspruch 19 oder 20, wobei die Einfüllöffnung nach dem Füllen durch die eine Folie (1,2) verschlossen bleibt.

22. Medikamentenmagazin nach einem der Ansprüche 1-4, wobei die Innenstruktur (3) als Stech- und Zentrierhilfe für äussere Saug- und Stechmittel ausgebildet ist.

23. Medikamentenmagazin nach Anspruch 22, wobei die Innenstruktur (3) ein zweiseitiges äusseres Anstechen von gegenüberliegenden Seiten erlaubt.

24. Medikamentenmagazin nach Anspruch 22 oder 23, wobei in die Innenstruktur (3) ein Filter (15) oder Sieb (18) integriert ist.

25. Medikamentenmagazin nach Anspruch 1 und 2, wobei die Innenstruktur (3) Mittel zum Öffnen und Stabilisieren der Medikamentenkammer aufweist.

26. Medikamentenmagazin nach einem der vorhergehenden Ansprüche, mit 60 Medikamentenkammern, welche 60 Medikamenten-Einzeldosen entsprechen.

27. Verwendung des Medikamentenmagazins nach einem der vorhergehenden Ansprüche in einem Mehrdosispulverinhalator.

28. Mehrdosispulverinhalator mit einem Medikamentenmagazin nach einem der Ansprüche 1-26.

29. Mehrdosispulverinhalator nach Anspruch 28 zur Applikation eines Arzneimittels, das einen Wirkstoff oder eine Kombination der Wirkstoffe enthält ausgewählt aus der Gruppe Betamimetika, Anticholinergika, Steroide, Antiallergika, Derivate von Mutterkornalkaloiden, Triptane, CGRP-Antagonisten, der Phosphodiesterase -V-Inhibitoren, Phosphodiesterase-IV-Inhibitoren, LTD4-Antagonisten, EGFR-Kinase-Hemmer.
